(19) Europäisches Patentamt
European Patent Office
Office européen des brevets

(11) Veröffentlichungsnummer: **0 051 802 B1**

(12) **EUROPÄISCHE PATENTSCHRIFT**

(45) Veröffentlichungstag der Patentschrift: **23.01.85**

(51) Int. Cl.⁴: **C 07 C 85/11**, C 07 C 87/50, C 07 C 87/56

(21) Anmeldenummer: **81109097.6**

(22) Anmeldetag: **28.10.81**

(54) **Verfahren zur Herstellung von aromatischen Aminen.**

(30) Priorität: **06.11.80 DE 3041892**

(43) Veröffentlichungstag der Anmeldung: **19.05.82 Patentblatt 82/20**

(45) Bekanntmachung des Hinweises auf die Patenterteilung: **23.01.85 Patentblatt 85/4**

(84) Benannte Vertragsstaaten: **CH DE FR GB LI**

(56) Entgegenhaltungen:
**DE - B - 2 331 878**

**HOUBEN-WEYL "Methoden der organischen Chemie", 4. Auflage, Band XI/1, 1957, GEORG THIEME VERLAG, Stuttgart, Seite 537**

(73) Patentinhaber: **HOECHST AKTIENGESELLSCHAFT, Postfach 80 03 20, D-6230 Frankfurt am Main 80 (DE)**

(72) Erfinder: **Müller, Werner Heinrich, Dr., Taunusblick 5, D-6239 Eppstein/Taunus (DE)**
Erfinder: **Berthold, Rüdiger, Dr., Geierfeld 55, D-6232 Bad Soden am Taunus (DE)**

EP 0 051 802 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der vorliegenden Erfindung ist ein Verfahren zur Herstellung von aromatischen Aminen durch Umsetzung von Cyclohexanon- bzw. Cyclohexenonazinen in der Gasphase an Edelmetall der 8. Nebengruppe des Periodensystems enthaltenden Katalysatoren.

Aromatische Amine sind vielseitig verwendbare Zwischenprodukte, z. B. für Farbstoffe, Arzneimittel oder Pflanzenschutzpräparate.

Bisher stellt man aromatische Amine hauptsächlich durch Reduktion der entsprechenden aromatischen Nitroverbindungen oder durch Umsetzung von entsprechenden Phenolen mit Ammoniak bei hohen Temperaturen und Drücken her.

Voraussetzung für diese Verfahren ist also die Verfügbarkeit der entsprechenden Nitroverbindungen bzw. Phenole. Da bei der Nitrierung von Aromaten immer verschiedene Isomere anfallen, ergibt sich oft ein schwieriges Trennproblem, das entweder vor der Reduktion, d. h. auf der Stufe der Nitroverbindungen, oder nach der Reduktion, d. h. auf der Aminstufe, gelöst werden muß. Die Umsetzung der Phenole mit Ammoniak bedingt einen sehr großen technischen und apparativen Aufwand. Außerdem müssen diese Phenole häufig aus Steinkohlenteer gewonnen werden, beispielsweise das sym. m-Xylenol.

Ziel der vorliegenden Erfindung war es daher, eine von aromatischen Vorstufen unabhängige Herstellung aromatischer Amine zu finden, und zwar ausgehend von einfachen aliphatischen Grundstoffen wie z. B. cycloaliphatischen Ketonen.

Cyclische Ketone, beispielsweise Cyclohexanon, werden in großem Maßstab durch Oxidation von Cycloaliphaten hergestellt. Ungesättigte cyclische Ketone, beispielsweise 3,5-Dialkylcyclohexanone, sind aus Aldehyden und γ-Ketoestern leicht zugänglich (vgl. DE-OS 2 654 850).

Nach E. C. Horning (J. Am. Chem. Soc., 69, 1907 (1947)) gelingt die Überführung von Cyclohexenon in Anilin durch Umsetzung des Cyclohexenons mit Hydrazin und katalytische Dehydrierung des dabei gebildeten Cyclohexenonazins an Pd/C-Katalysatoren in der Flüssigphase bei Temperaturen zwischen 136°C und 252°C:

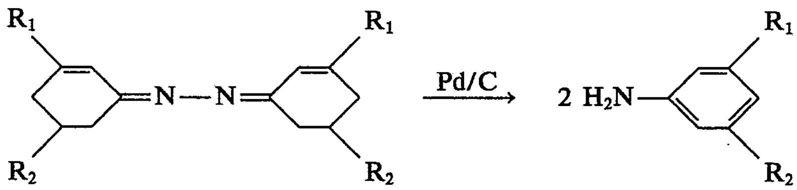


Eine wirtschaftliche Herstellung aromatischer Amine nach diesem Verfahren ist jedoch wegen der nur etwa 50% erreichenden Ausbeuten nicht möglich.

Es wurde nun ein Verfahren zur Herstellung von aromatischen Aminen der allgemeinen Formel Ia bzw. Ib

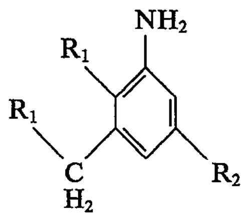

(Ia)

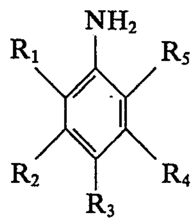

(Ib)

gefunden, wobei die Reste $R_1$ bis $R_5$ Wasserstoff oder $C_1-C_6$-Alkylgruppen oder einer der Reste eine Phenylgruppe sein können, dadurch gekennzeichnet, daß man ein Cyclohexenon-azin bzw. Cyclohexanon-azin der allgemeinen Formel IIa bzw. IIb

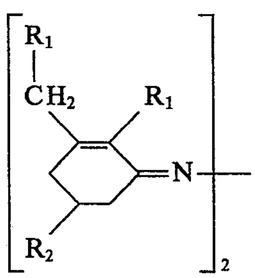

(IIa)

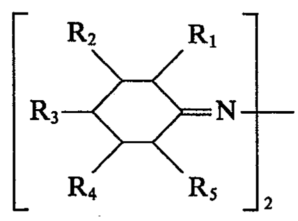

(IIb)

worin die Reste $R_1$ bis $R_5$ die oben genannte Bedeutung haben, in der Gasphase bei Temperaturen von 200 bis 500°C über einen mindestens ein Edelmetall der 8. Nebengruppe des Periodensystems enthaltenden Katalysator leitet.

Die Verbindungen der Formel IIa können auf folgende Weise (DE-OS 2 654 850; Ann. 281, 104 (1894) hergestellt werden:

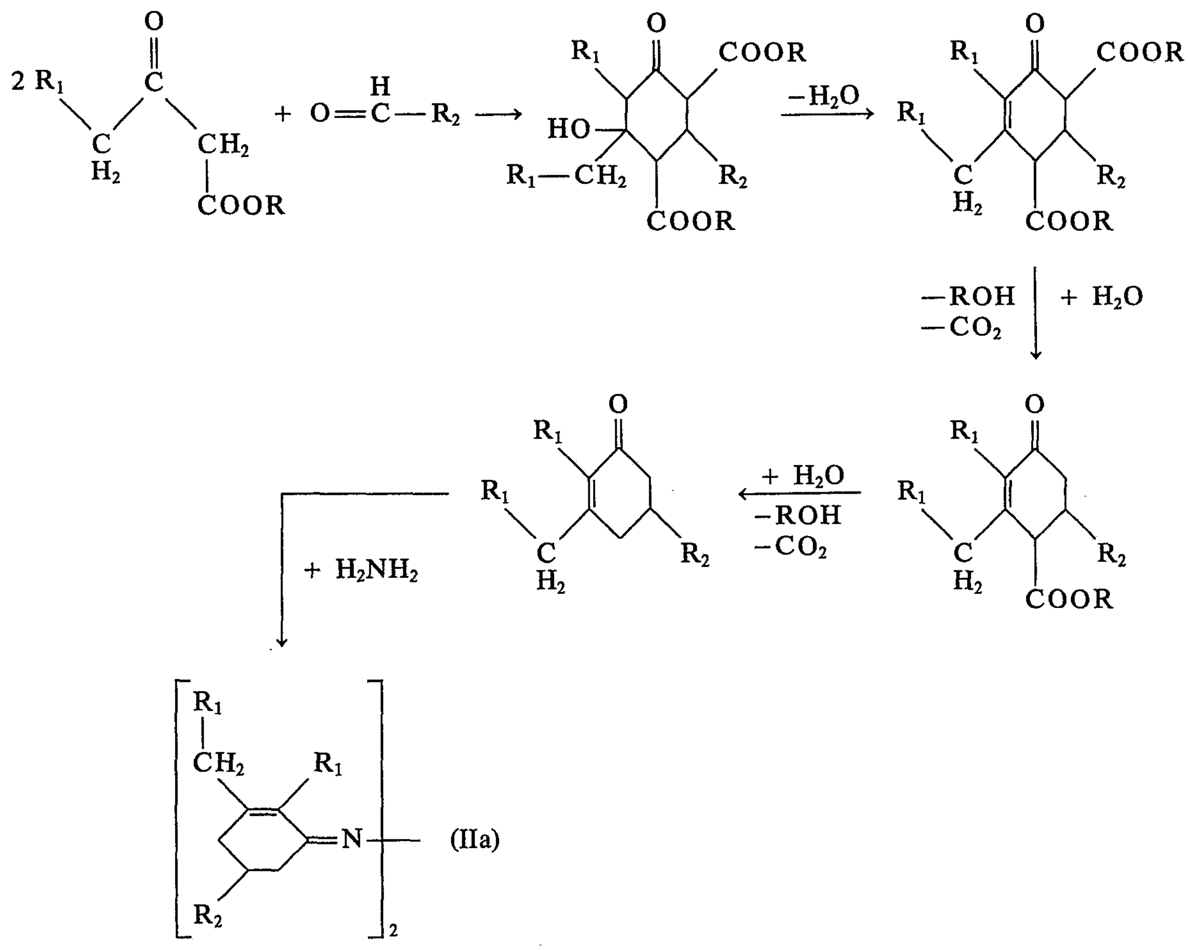


(IIa)

Die Cyclohexanonazine der Formel IIb werden aus cycloaliphatischen Ketonen, z. B. Cyclohexanon, Methylcyclohexanon und Hydrazin hergestellt.

Im allgemeinen haben die Reste $R_1$ bis $R_5$ zusammen nicht mehr als 12-C-Atome. Die für $R_1$ bis $R_5$ in Frage kommenden $C_1-C_6$-Alkylreste können geradkettig, verzweigt oder cyclisch sein. Einer der Reste $R_1$ bis $R_5$ kann auch ein Phenylrest sein. Dieser kann auch durch Halogeno-, $C_1-C_6$-Alkyl- oder $C_1-C_6$- Alkoxy-Gruppen substituiert sein. Vorzugsweise ist er aber unsubstituiert oder nur einfach substituiert.

Zur erfindungsgemäßen Umsetzung der Azine in Amine verwendet man im allgemeinen Katalysatoren, die Ruthenium, Rhodium, Palladium, Iridium oder Platin oder deren Gemische enthalten. Palladium, Platin und Palladium/Platin sind bevorzugt.

Im allgemeinen werden geträgerte Katalysatoren, beispielsweise mit Kohle, $SiO_2$, $Al_2O_3$, Aluminiumsilikaten, Spinellen, Chromoxid-Aluminiumoxid und Zeolithen als Trägern, verwendet. Die Konzentration des Edelmetalls auf dem Träger beträgt dabei im allgemeinen 0,05 bis 10 Gew.-%, vorzugsweise 0,2 bis 5 Gew.-%, insbesondere 0,3 bis 2,5 Gew.-%, bezogen auf das Gewicht des Trägers. Die Edelmetalle werden als Verbindungen auf den Träger aufgebracht und vorzugsweise vor Beginn der Reaktion reduziert, z. B. durch Überleiten von Wasserstoff. Der Katalysator kann entweder als Festbett, als bewegtes Bett oder als Wirbelschicht angeordnet sein.

Die Reaktionstemperatur beträgt im allgemeinen 200—500°C, vorzugsweise 250—400°C, insbesondere 270—380°C.

Im allgemeinen wird Unterdruck bis herab zu etwa 10 mbar oder Normaldruck angewandt, man kann jedoch auch bei höheren Drucken bis etwa 20 bar arbeiten. Vorzugsweise verwendet man ein Trägergas, um das Azin über den Katalysator zu transportieren. Als Trägergas eignen sich besonders Wasserstoff, Stickstoff, Ammoniak, Argon, $CO_2$, Methan, Wasserdampf, Ethylen und Propylen.

Das Azin kann vor dem Reaktor auch mit leichtflüchtigen Lösemitteln, wie z. B. Kohlenwasserstoffen, Ethern, insbesondere Glykol- und Polyglykol-dialkylethern oder Wasser verdünnt werden.

Die folgenden Beispiele dienen zur Erläuterung des erfindungsgemäßen Verfahrens. Die angegebenen Litermengen an $N_2$ und $H_2$ beziehen sich stets auf den Normzustand (0°C, 1,013 bar).

Beispiel 1

In einem Glasreaktor von 18 mm Durchmesser und 40 cm Länge befanden sich von oben nach unten zuerst eine 5 cm lange Schicht aus Glaskugeln und unmittelbar danach eine 16 cm lange Schicht eines Katalysators, bestehend aus 1 Gew.-% Pd auf $SiO_2$-Kugeln. Der Reaktor wurde im $N_2$-Strom auf 300°C aufgeheizt, anschließend wurde der Katalysator mit 24 l Stickstoff und 6 l Wasserstoff pro Stunde 2 Stunden lang bei 200°C aktiviert.

Daraufhin wurden in 2 Stunden 100 g einer Mischung, bestehend aus 80 g Dimethyldiglykol und 20 g 3,5-Dimethylcyclohexen-2-azin-1 ($Kp_{0.5}$ 106°C) zusammen mit 40 l Stickstoff und 28 l Wasserstoff von oben über den durch eine elektrische Heizung auf 280°C erhitzten Katalysator geleitet. Am Reaktorausgang wurden während der 2. Stunde 48 g Produkt aufgefangen. Die gaschromatographische Analyse (GC) ergab 7,7 g 3,5-Dimethylanilin, d.h. 78% d.Th., und 1,2 g Dixylylamin ≙ 13% d.Th.

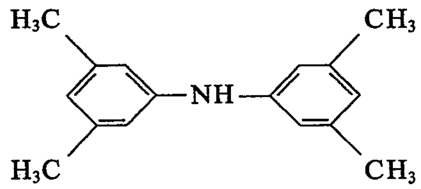

Dixylylamin

Beispiel 2

Mit Hilfe des in Beispiel 1 beschriebenen Reaktors und Katalysators wurde bei 300°C 3 Stunden lang ein Gemisch aus 20 g 3-Methyl-5-propyl-cyclohexen-2-onazin-1 ($Kp_{0.5}$ 198—200°C) und 80 g Toluol umgesetzt. Gleichzeitig wurden in dieser Zeit 60 l $N_2$ und 30 l $H_2$ über den Katalysator geleitet. Das am Reaktorende kondensierte Produkt enthielt laut GC-Analyse 14 g 3-Methyl-5-propylanilin ≙ 70% d.Th. ($Kp_1$ 106°C).

Beispiel 3

Mit Hilfe des in Beispiel 1 beschriebenen Reaktors und Katalysators wurde bei 270 bis 280°C 2 Stunden lang eine Mischung von 20 g Cyclohexanonazin und 80 g Di-n-butylether umgesetzt. Während dieser Zeit wurden ebenfalls 40 l $N_2$ und 28 l $H_2$ über den Katalysator geleitet. Das am Reaktorausgang aufgefangene Produkt enthielt laut GC-Analyse 15,1 g Anilin, 2,7 g Diphenylamin und 1,0 g umgesetztes Cyclohexanonazin.

**Patentansprüche**

1. Verfahren zur Herstellung von aromatischen Aminen der allgemeinen Formeln Ia bzw. Ib.

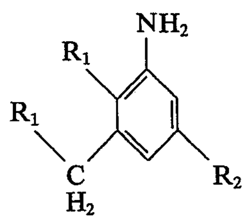

(Ia)

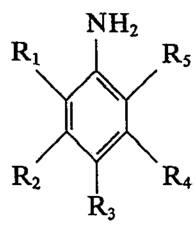

(Ib)

wobei die Reste $R_1$ bis $R_5$ Wasserstoff oder $C_1$—$C_6$-Alkylgruppen sein können oder einer der Reste $R_1$ bis $R_5$ eine Phenylgruppe oder eine durch Halogen, $C_1$—$C_6$-Alkyl- oder $C_1$—$C_6$-Alkoxygruppen substituierte Phenylgruppe sein kann, dadurch gekennzeichnet, daß man ein Cyclohexenon-azin bzw. Cyclohexanon-azin der allgemeinen Formel IIa bzw. IIb

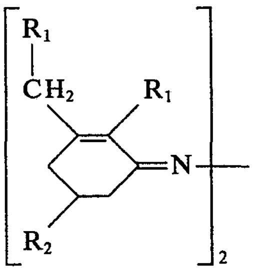

(IIa)

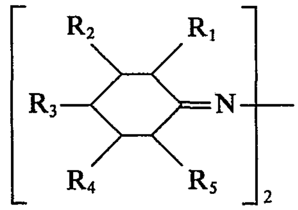

(IIb)

worin die Reste $R_1$ bis $R_5$ die oben genannte Bedeutung haben, in der Gasphase bei Temperaturen von 200 bis 500°C über einen mindestens ein Edelmetall der 8. Nebengruppe des Periodensystems enthaltenden Katalysator leitet.

2. Verfahren nach Anspruch 1, dadurch gekennzeichnet, daß als Edelmetall Palladium oder Platin oder deren Gemisch eingesetzt wird.

## Claims

1. A process for the preparation of aromatic amines of the general formula Ia or Ib

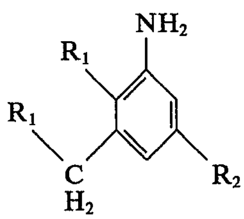

(Ia)

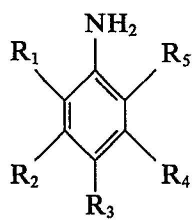

(Ib)

in which $R_1$ through $R_5$ each are hydrogen or $C_1$—$C_6$-alkyl, or one of these radicals $R_1$ through $R_5$ is phenyl or phenyl substituted by halogen, $C_1$—$C_6$-alkyl or $C_1$—$C_6$-alkoxy, characterized by passing a cyclohexenone azine or cyclohexanone azine of the general formula IIa or IIb

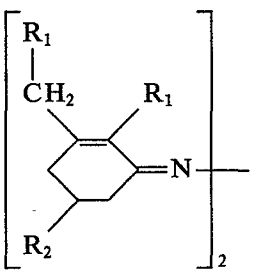

(IIa)

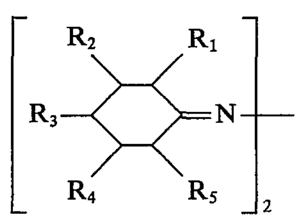

(IIb)

in which $R_1$ through $R_5$ are as defined above, in the gaseous phase, at a temperature of from 200 to 500°C, over a catalyst containing at least one noble metal of the 8th subgroup of the Periodic Table.

2. The process as claimed in Claim 1, wherein palladium or platinum or a mixture thereof is used as noble metal.

**Revendications**

1. Procédé pour la préparation d'amines aromatiques de formules générales Ia ou Ib

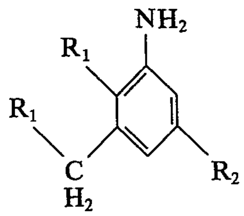

(Ia)

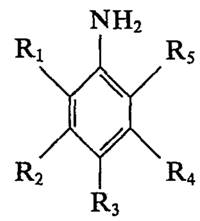

(Ib)

où les radicaux $R_1$ à $R_5$ peuvent être l'hydrogène ou des groupes alkyle à 1 à 6 atomes de carbone, ou l'un des radicaux $R_1$ à $R_5$ peuvent être un groupe phényle ou un groupe phényle substitué par un halogène ou un groupe alkyle à 1 à 6 atomes de carbone ou alcoxy à 1 à 6 atomes de carbone, caractérisé en ce qu'on fait passer en phase gazeuse, à des températures de 200—500°C, sur un catalyseur contenant au moins un métal noble de 8ème groupe du tableau périodique, une cyclohexénone-azine ou une cyclohexanone-azine de formule générale IIa ou IIb

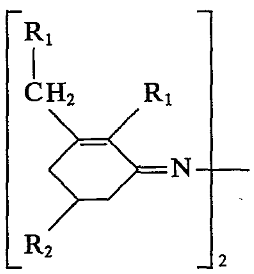

(IIa)

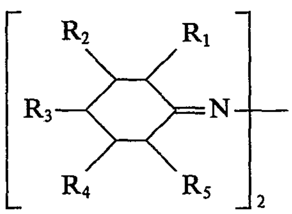

(IIb)

où les radicaux $R_1$ à $R_5$ ont la signification donnée ci-dessus.

2. Procédé selon la revendication 1, caractérisé en ce qu'on utilise en tant que métal noble du palladium, ou du platine, ou leurs mélanges.